# EUROPÄISCHE PATENTSCHRIFT

(11) **EP 2 049 598 B1**
(45) Veröffentlichungstag und Bekanntmachung des Hinweises auf die Patenterteilung: **21.07.2010**
(21) Anmeldenummer: 07703815.6
(22) Anmeldetag: 12.01.2007
(51) Int. Cl.: C08L 97/02, B27N 3/00, F24F 5/00, A61L 9/00

(54) **VERWENDUNG VON HOLZWERKSTOFFEN ENTHALTEND POLYAMIN ZUR SENKUNG DES FORMALDEHYDGEHALTS IN DER UMGEBUNGSLUFT**
USE OF TIMBER MATERIALS COMPRISING POLYAMINE FOR LOWERING FORMALDEHYDE CONTENT IN AMBIENT AIR
UTILISATION DE MATÉRIAUX DÉRIVÉS DE BOIS CONTENANT DE LA POLYAMINE POUR DIMINUER LA TENEUR EN FORMALDÉHYDE DE L'AIR AMBIANT

(30) Priorität: 27.07.2006 EP 06118012
(43) Veröffentlichungstag der Anmeldung: 22.04.2009
(73) Patentinhaber: BASF SE, 67056 Ludwigshafen (DE)
(72) Erfinder: WEINKÖTZ, Stephan, 67434 Neustadt (DE); BEIL, Christian, 67551 Worms (DE); FINKENAUER, Michael, 67593 Westhofen (DE); RÜBA, Eva, 8008 Zürich (CH); SCHMIDT, Michael, 67346 Speyer (DE)
(86) Internationale Anmeldenummer: PCT/EP2007/050273
(87) Internationale Veröffentlichungsnummer: WO 2008/012113

(56) Entgegenhaltungen:
- DE-A1- 19 930 525
- US-A- 4 547 350
- US-A- 4 892 719
- DATABASE WPI Week 199717 Derwent Publications Ltd., London, GB; AN 1997-187301 XP002462940 & JP 09 047623 A (TOYOBO KK) 18. Februar 1997 (1997-02-18) & DATABASE CA CHEMICAL ABSTRACTS SERVICE, COLUMBUS, OHIO, US; "Aldehyde adsorbing deodorization sheets and their manufacture" gefunden im STN Database accession no. 126:242064
- DATABASE WPI Week 200215 Derwent Publications Ltd., London, GB; AN 2002-109769 XP002462941 & JP 2001 293075 A (TEIJIN LTD) 23. Oktober 2001 (2001-10-23) & DATABASE CA CHEMICAL ABSTRACTS SERVICE, COLUMBUS, OHIO, US; "Artificial ornamental plant having formaldehyde-deodorizing and -absorbing ability" gefunden im STN Database accession no. 135:334567
- DATABASE WPI Week 200273 Derwent Publications Ltd., London, GB; AN 2002-676873 XP002463016 & JP 2002 119852 A (MITSUBISHI CHEM CORP) 23. April 2002 (2002-04-23)

## Beschreibung

Die vorliegende Erfindung betrifft die Verwendung von Holzwerkstoffen zur Herstellung von Möbelteilen, Wandverkleidungen, Isolationsmaterialen und dergleichen zur Senkung des Formaldehydgehalts in der Umgebungsluft, wobei die Holzwerkstoffe
(i) Polyamin als Bindemittel enthalten oder
(ii) ein von Polyamin verschiedenes Bindemittel enthalten und zusätzlich in oder auf den äußeren Schichten des Holzwerkstoffs Polyamin aufweisen
und das Polyamin ein Molekulargewicht von mindestens 500 g/mol und mindestens 6 primäre oder sekundäre Aminogruppen aufweist.

Zur Herstellung von Holzwerkstoffen werden seit langem Kondensationsprodukte auf der Basis von Harnstoff, gegebenenfalls Melamin, und Formaldehyd verwendet. Diese werden hauptsächlich zur Herstellung von Faser- oder Spanplatten für den Möbelbau eingesetzt. Neben ihrem günstigen Preisniveau besitzen diese Harze die Vorteile der einfachen Verarbeitbarkeit und langer Tropfzeiten bei gleichzeitig hohen Reaktivitäten. Der gravierende Nachteil ist allerdings, dass diese Holzwerkstoffe bei und nach der Verarbeitung Formaldehyd abspalten.

Auch Holz selbst kann Formaldehyd an die Umgebung abgeben, insbesondere nach einer Wärmebehandlung. Beschichtete Holzwerkstoffe weisen im Allgemeinen eine geringere Formaldehyd-Emissionen auf als unbeschichtete Substrate ("Holz als Roh und Werkstoff" Band 47, 1989, Seite 227).

Formaldehyd kann oberhalb bestimmter Grenzwerte beim Menschen Allergien, Haut-, Atemwegs- oder Augenreizungen verursachen. Die Reduzierung von Formaldehyd im Innen- bzw. Wohnbereich ist daher ein wichtiges Anliegen.

DE 43 08 089 A1 beschreibt ein Bindemittel zur Holzverleimung enthaltend a) ein Polyamin, b) 0,01 bis 0,25 Mol pro Mol Aminogruppe von a) eines Zuckers und c) 0,01 bis 0,25 Mol pro Mol Aminogruppe von a) einer oder mehrerer Komponenten aus der Gruppe, die von Dicarbonsäurederivaten, Aldehyden mit zwei oder mehr Kohlenstoffatomen und Epoxiden gebildet wird. Als Polyamin wird beispielsweise Polyethylenimin oder N,N',N"-Tris(6-aminohexyl)melamin genannt. In den Beispielen wird eine Formaldehydemission von 0,04 bis 0,1 mg HCHO /m²h beschrieben.

EP 1 192 223 B1 beschreibt eine Faserplatte aus Polyaminen oder Polyamin-haltigen Aminoplastharzen als Bindemittel. Als Leimlösung wird unter anderen eine wässerige Lösung eines aliphatischen Polyamins mit mindestens drei funktionellen Gruppen, ausgewählt aus der Gruppe der primären und sekundären Aminogruppen genannt, welches ein gewichtsmittleres Molgewicht von 600 bis 1000000 g/mol aufweist und abgesehen von tertiären Aminogruppen im wesentlichen frei von sonstigen funktionellen Gruppen ist. Es wird beschrieben, dass als bevorzugtes Polyamin Polyethylenimin oder Polyvinylamin eingesetzt wird. Es wird offenbart, dass das Polyethylenimin bevorzugt ein gewichtsmittleres Molgewicht von 800 bis 100000 und das Polyvinylamin bevorzugt ein gewichtsmittleres Molgewicht von 5000 bis 200000 aufweist.

Im Stand der Technik finden sich demnach mehrere Hinweise, wie die genannten formaldehydhaltige Bindemittel ersetzt werden können. Es gibt allerdings im Wohnbereich weitere Formaldehydquellen, wie beispielsweise Textilien, Spanplatten, Möbel, insbesondere ältere Möbel, und Zigarettenrauch.

JP 2001 293075 A (TEIJIN LTD) offenbart die Verwendung von einer Kunstpflanze zur Senkung des Formaldehydgehalts in der Umgebungsluft. Die aus Kunstfasern gemachte Blätter der Kunstpflanze enthalten eine Aminoverbindung als Formaldehydadsorber (Polyvinylamin wird erwähnt).

JP 09 047623 A (TOYOBO KK) offenbart ein Aktivkohle enthaltendes adsorbtives Blatt aus Fasermaterial (Hanf, Holzpappe..., siehe Absatz 0011), das mit einer Zusammensetzung aus aliphatischer Aminoverbindung und Säure beschichtet wird. Als Aminoverbindung wird Polyethylenimine genannt (Absatz 0015). Das Blatt ist im Autos und im Innenbereich von Häusern zur Senkung des Aldehydgehalts in der Umgebungsluft anwendbar.

CA 1 241 524 beschreibt die Verwendung von Polyaminen als Formaldehydfänger. Beispielsweise werden Filter in Heizsystemen mit den Polyaminen beschichtet oder die Polyamine werden als Additive Wandfarben zugegeben.

Die Aufgabe der vorliegenden Erfindung bestand darin, Holzwerkstoffe aufzuzeigen, die Formaldehyd aus der Umgebungsluft aufnehmen können, wobei diese Holzwerkstoffe bereits im Wohnbereich Verwendung finden, bzw. zweckmäßig in den Wohnbereich integrierbar sind.

Die Aufgabe wurde gelöst unter Verwendung von Holzwerkstoffen zur Herstellung von Möbelteilen, Wandverkleidungen, Isolationsmaterialen und dergleichen zur Senkung des Formaldehydgehalts in der Umgebungsluft, wobei die Holzwerkstoffe
(i) Polyamin als Bindemittel enthalten oder
(ii) ein von Polyamin verschiedenes Bindemittel enthalten und zusätzlich in oder auf den äußeren Schichten des Holzwerkstoffs Polyamin aufweisen
und das Polyamin ein Molekulargewicht von mindestens 500 g/mol und mindestens 6 primäre oder sekundäre Aminogruppen aufweist.

Bevorzugt werden Polyamine eingesetzt, die ein Molekulargewicht von mindestens 800 g/mol und mindestens 6, insbesondere mindestens 10, primäre oder sekundäre Aminogruppen aufweisen. Es kann nur ein bestimmtes Polyamin oder Mischungen aus mehreren Polyaminen verwendet werden. Als Polyamine werden bevorzugt Polyethylenimin oder Polyvinylamin oder Mischungen hiervon gewählt.

Das gewichtsmittlere Molekulargewicht des Polyvinylamins beträgt vorteilhaft 5.000 bis 500.000 g/mol, bevorzugt 5.000 bis 350.000 g/mol, insbesondere 5.000 bis 100.000. Das gewichtsmittlere Molekulargewicht des Polyethylenimins beträgt vorteilhaft 500 bis 100.000 g/mol, bevorzugt 500 bis 70.000 g/mol, besonders bevorzugt 500 bis 50.000 g/mol und insbesondere 500 bis 20.000 g/mol.

Zur Verwendung des Polyamins als Bindemittel gemäß Fall (i):
Das Polyamin wird vorteilhaft in Form von wässrigen Lösungen mit einem Feststoffgehalt an Polyamin von 1 bis 95 Gew.-%, bevorzugt 5 bis 80 Gew.-% als Bindemittel eingesetzt. Im Fall einer wässrigen Polyvinylamin-Lösung liegt der Feststoffgehalt bevorzugt bei 5 bis 30 Gew.-%, insbesondere bei 5 bis 15 Gew.-%. Im Fall einer wässrigen Polyethylenimin-Lösung liegt der Feststoffgehalt bevorzugt bei 10 bis 60 Gew.-%, insbesondere bei 30 bis 50 Gew.-%.

Die Polyaminlösung kann übliche Hilfs- und Zusatzstoffe wie Hydrophobiermittel, z.B. Paraffine, Holzschutzmittel oder Flammschutzmittel enthalten.

Die Polyaminlösung wird nach üblichen Verfahren auf cellulosehaltige Späne/Fasern aufgebracht (vgl. "MDF - Mitteldichte Faserplatten", Hans-Joachim Deppe, Kurt Ernst, 1996, DRW-Verlag Weinbrenner GmbH & Co., 70771 Leinfelden-Echterdingen, Kapitel 4.3, Seiten 81ff.; siehe auch EP 1 192 223 B1, Paragraph [0034]).

Die Polyaminlösung wird als Bindemittel vorteilhaft in solchen Mengen eingesetzt, dass pro 100 g atro Fasern/Späne 0,1 bis 20 g, bevorzugt 0,2 bis 5 g, besonders bevorzugt 0,5 bis 2,5 g Polyamin verwendet werden.

Anschließend werden die cellulosehaltigen Späne oder Fasern nach einem üblichen Verfahren zu Holzwerkstoffen verpresst. Hierzu erzeugt man durch Aufstreuen der cellulosehaltigen Späne oder Fasern auf einen Träger eine Späne- oder Fasermatte und verpresst diese(n) bei Temperaturen von 80 bis 250°C und bei Drücken von 5 bis 50 bar zu Holzwerkstoffen (vgl. loc. cit. Kapitel 4.5, Seiten 93ff).

Bevorzugt werden die cellulosehaltigen Fasern zu Holzwerkstoffen einer solchen Schichtdicke aufgestreut, dass nach dem Heißverpressen Holzwerkstoffe mit einer Dichte von 100 bis 1000 kg/m³, bevorzugt 450 bis 900 kg/m³, und einer Dicke von 0,5 bis 200 mm, bevorzugt 1 bis 40 mm, besonders bevorzugt 1,5 bis 20 mm, resultieren.

Zur Verwendung des Polyamins in einer Holzwerkstoff-Schutzschicht gemäß Fall (ii):
Die Polyamine oder die wässrige Polymerlösung können auf den verleimten Holzwerkstoff aufgebracht werden. Als Bindemittel können alle dem Fachmann bekannten Bindemittel zur Holzwerkstoff-Herstellung verwendet werden, insbesondere Aminoplastharze.

Die wässrige Polymerlösung ist insbesondere erhältlich durch Mischen von - jeweils bezogen auf die Polymerlösung -
(a) 1 bis 99 Gew.-% Polyamin
(b) 0 bis 5 Gew.-% Additiven zur Verbesserung der Benetzbarkeit
(c) 0 bis 30 Gew.-% Additiven zur Einstellung des pH-Wertes
(d) 0 bis 30 Gew.-% anderen Additiven wie Fungiziden, Hydrophobierungsmitteln, Farbstoffen, organischen Lösungsmitteln
(e) 0 bis 20 Gew.-% Harnstoff
und ergänzend zu 100 Gew.-% Wasser, wobei sich diese Angaben auf den Beginn des Mischens beziehen.

Die wässrige Polyaminlösung enthält vorteilhaft 5 bis 90 Gew.-% Polyamin, bevorzugt 10 bis 75 Gew.-% Polyamin, insbesondere 15 bis 45 Gew.-% Polyamin, besonders bevorzugt 25 bis 40 Gew.-% Polyamin, jeweils bezogen auf die Polymerlösung.

Als fakultative Komponente (b) der wässrigen Polymerlösung können zur Verbesserung der Benetzbarkeit ionische und nichtionische Tenside, wie sie beispielsweise in H. Stache, "Tensid-Taschenbuch", Carl Hanser Verlag, München, Wien, 1981, beschrieben sind, in Konzentration von vorteilhaft 0 bis 5 Gew.-%, bevorzugt von 0 bis 2 Gew.-%, eingesetzt werden.

Der pH-Wert lässt sich durch die folgenden Additive als fakultative Komponente (c) einstellen: Mineralische oder organische Säuren wie beispielsweise Schwefelsäure oder Ameisensäure. Komponente (c) kann der wässrigen Polymerlösung von 0 bis 30 Gew.-%, bevorzugt von 0 bis 20 Gew.-%, zugegeben werden.

Als fakultative Komponente (d) können weitere Additive der wässrigen Polymerlösung hinzugefügt werden, beispielsweise Additive aus der Gruppe Hydrophobierungsmittel, z. B. Paraffinemulsionen und Wachse, Fungizide, organische Lösungsmittel oder Farbstoffe. Komponente (d) kann der wässrigen Polymerlösung von 0 bis 30 Gew.-%, bevorzugt von 0 bis 10 Gew.-%, zugegeben werden.

Als fakultative Komponente (e) kann die wässrige Polyaminlösung bis zu 20 Gew.-% Harnstoff, bezogen auf die Polymerlösung, enthalten. Vorteilhaft enthält die wässrige Polymerlösung weniger als 15 Gew.-% Harnstoff, bevorzugt weniger als 10 Gew.-% Harnstoff und insbesondere weniger als 5 Gew.-% Harnstoff, jeweils bezogen auf die Polymerlösung. Besonders bevorzugt ist die Polymerlösung frei von Harnstoff.

Die Polyaminlösung wird vorteilhaft mit einem pH-Wert von 3 bis 12, bevorzugt von 6 bis 11, insbesondere mit einem pH-Wert von 9 bis 11 aufgebracht.

Es wird eine ausreichende Menge an Polyaminlösung aufgebracht, so dass die Menge an Polyamin pro m² Oberfläche des Holzwerkstoffs zwischen 0,1 g und 100 g, bevorzugt zwischen 0,5 g und 50 g, besonders bevorzugt zwischen 1 g und 10 g beträgt. Die wässrige Polymerlösung kann durch verschiedene, dem Fachmann bekannte Maßnahmen auf den Holzwerkstoff aufgebracht werden. Dazu zählen beispielsweise Aufsprühen, Walzen, Tauchen, Rakeln, Streichen oder Curtain Coating. Bevorzugt wird die Aminlösung durch Aufsprühen und Aufwalzen, insbesondere durch Aufwalzen, aufgebracht.

Die nach dem Verfahren (i) oder (ii) erhaltenen Holzwerkstoffe können wie üblich weiterverarbeitet werden und eignen sich insbesondere für die Herstellung von Möbelteilen, Wandverkleidungen, Isolationsmaterialen und dergleichen, d.h. Gegenstände/Objekte aus Holz, die in direktem Kontakt zur Raumluft oder zu einer Formaldehydquelle stehen. Bevorzugt werden die so hergestellten Holzwerkstoffe zur Herstellung von Möbelrückwänden verwendet.

Bevorzugt werden die Holzwerkstoffe nach dem Verfahren (i) erhalten und sind somit formaldehydfrei.

Die so hergestellten Möbelteile, Wandverkleidungen, Isolationsmaterialen und dergleichen stellen selbst nicht nur formaldehydfreie, bzw. formaldehydarme Gegenstände dar, sondern diese Gegenstände können auch noch Formaldehyd aus der Umgebungsluft aufnehmen und reduzieren somit dauerhaft die Formaldehydbelastung in beispielsweise Wohnräumen.

### Beispiele

### Beispiel 1:

Es wurde eine MDF-Platte A (30 x 30 cm), die eine Dichte von 850 kg/m³ und eine Dicke von 4 mm aufwies, unter Verwendung eines Bindemittels bestehend aus einer 30 Gew.-% wässrigen Polyethylenimin-Lösung (gewichtsmittleres Molekulargewicht des Polyethylenimins: 5000 g/mol) hergestellt, wobei das Bindemittel in einer Menge von 1,5 g Feststoff pro 100 g atro Fasern verwendet wurde. Das Verpressen wurde bei einem Pressdruck von 4 N/mm², einer Presstemperatur von 200°C und einer Presszeit von 120 s durchgeführt.

Die Dicke und Dichte der MDF-Platte wurde entsprechend der üblichen Form zur Herstellung von beispielsweise Möbelrückwänden gewählt.

### Beispiel 2:

Es wurde eine MDF-Platte B (30 x 30 cm), die eine Dichte von 850 kg/m³ und eine Dicke von 4 mm aufwies, unter Verwendung eines Bindemittels bestehend aus einer 30 Gew.-% wässrigen Polyethylenimin-Lösung (gewichtsmittleres Molekulargewicht des Polyethylenimins: 5000 g/mol) hergestellt, wobei das Bindemittel in einer Menge von 3 g Feststoff pro 100 g atro Fasern verwendet wurde. Das Verpressen wurde analog zu Beispiel 1 durchgeführt.

### Beispiel 3:

Es wurde eine MDF-Platte C (30 x 30 cm), die eine Dichte von 850 kg/m³ und eine Dicke von 4 mm aufwies, unter Verwendung eines Harnstoff-Formaldehyd-Kondensationsharzes (Kaurit Leim 340, 68% Festharzgehalt) hergestellt, wobei das Bindemittel in einer Menge von 12 g Festharz pro 100 g atro Fasern verwendet wurde. Das Verpressen wurde analog zu Beispiel 1 durchgeführt. Anschließend wurde die Platte auf der Ober- und Unterseite mit 10 g/m² einer 30 Gew.-%igen Polyethylenimin-Lösung (gewichtsmittleres Molekulargewicht des Polyethylenimins: 1300 g/mol) besprüht (Wirksubstanz: 3 g/m²) und 24 h bei Raumtemperatur getrocknet.

### Beispiel 4:

Es wurde eine MDF-Platte D (30 x 30 cm), die eine Dichte von 850 kg/m³ und Dicke von 4 mm aufwies, ohne Verwendung eines Bindemittels hergestellt, wobei der feuchte Faserkuchen (20% Feuchte) zu einer losen Platte bei einem Pressdruck von 4 N/mm², einer Presstemperatur von 200 °C und einer Presszeit von 200 s verpresst wurde.

### Beispiel 5: Handelsübliche Spanplatte als Formaldehydquelle

Hierbei handelt es sich um eine handelsübliche Spanplatte E, die eine Dichte von 670 kg/m³ und eine Dicke von 16 mm und eine FA-Emission von 1,16 mg/l (Exsikkator-methode) aufwies. Die Dicke und Dichte der Spanplatte wurde entsprechend der üblichen Form zur Möbelherstellung gewählt.

### Messung der Formaldehyd-Emission:

Die Formaldehydemission wurde mittels Exsikkator-Methode (JIS A 5908) bestimmt. Jede Exsikkator-Messung erfolgte mit 10 Prüfkörpern. Entweder wurden 10 Prüfkörper eines Plattentyps ( Platten A bis D) vermessen oder 5 Prüfkörper (Platte E) und 5 Prüfkörper (Platte A bzw. B. bzw. C bzw. D) vermessen. Die Exsikkatorwerte sind in der Tabelle 1 zusammengestellt.

**Tabelle 1: Übersicht über die Exsikkatorwerte**

| Prüfkörper | Exsikkatorwert [mg/l] |
|---|---|
| MDF-Platte A | < 0,01 |
| MDF-Platte B | < 0,01 |
| MDF-Platte C | 0,03 |
| MDF-Platte D | 0,12 |
| Spanplatte E | 1,16 |
| Spanplatte E + MDF-Platte A | 0,29 |
| Spanplatte E + MDF-Platte B | 0,21 |
| Spanplatte E + MDF-Platte C | 0,25 |
| Spanplatte E + MDF-Platte D | 0,80 |

Die Beispiele zeigen, dass der Formaldehyd, der durch die Spanplatte E an die Umgebungsluft abgegeben wurde, durch den Einsatz einer MDF-Platte A, B oder C gefangen wurde und sich somit der Formaldehydgehalt der Raumluft effektiv reduzieren lässt.

## Patentansprüche

1. Verwendung von Holzwerkstoffen zur Herstellung von Möbelteilen, Wandverkleidungen, Isolationsmaterialen und dergleichen zur Senkung des Formaldehydgehalts in der Umgebungsluft, wobei die Holzwerkstoffe
(i) Polyamin als Bindemittel enthalten oder
(ii) ein von Polyamin verschiedenes Bindemittel enthalten und zusätzlich in oder
auf den äußeren Schichten des Holzwerkstoffs Polyamin aufweisen und das Polyamin ein Molekulargewicht von mindestens 500 g/mol und mindestens 6 primäre oder sekundäre Aminogruppen aufweist.

2. Verwendung nach Anspruch 1, wobei das Polyamin ein Molekulargewicht von mindestens 800 g/mol und mindestens 10 primäre oder sekundäre Amino-Gruppen aufweist.

3. Verwendung nach Anspruch 1 oder 2, wobei als Polyamin Polyvinylamin und/oder Polyethylenimin eingesetzt wird.

4. Verwendung nach Anspruch 3, wobei das Polyvinylamin ein gewichtsmittleres Molekulargewicht von 5.000 bis 500.000 g/mol und das Polyethylenimin ein gewichtsmittleres Molekulargewicht von 500 bis 100.000 g/mol aufweist.

5. Verwendung nach Anspruch 3, wobei das Polyvinylamin ein gewichtsmittleres Molekulargewicht von 5.000 bis 100.000 g/mol und das Polyethylenimin ein gewichtsmittleres Molekulargewicht von 500 bis 20.000 g/mol aufweist.

6. Verwendung von Holzwerkstoffen nach den Ansprüchen 1 bis 5 zur Herstellung von Möbelrückwänden.

## Claims

1. The use of wood-base materials for the production of furniture parts, wall claddings, insulation materials and the like for reducing the formaldehyde content in the ambient air, the wood-base materials
(i) comprising polyamine as a binder or
(ii) comprising a binder differing from polyamine and additionally having polyamine in or on the outer layers of the wood-base material
and the polyamine having a molecular weight of at least 500 g/mol and at least 6 primary or secondary amino groups.

2. The use according to claim 1, the polyamine having a molecular weight of at least 800 g/mol and at least 10 primary or secondary amino groups.

3. The use according to claim 1 or 2, the polyamine used being polyvinylamine and/or polyethylenimine.

4. The use according to claim 3, the polyvinylamine having a weight average molecular weight of from 5000 to 500 000 g/mol and the polyethylenimine having a weight average molecular weight of from 500 to 100 000 g/mol.

5. The use according to claim 3, the polyvinylamine having a weight average molecular weight of from 5000 to 100 000 g/mol and the polyethylenimine having a weight average molecular weight of from 500 to 20 000 g/mol.

6. The use of wood-base materials according to claims 1 to 5 for the production of furniture rear panels.

## Revendications

1. Utilisation de matériaux à base de bois pour la fabrication de parties de meubles, de revêtements muraux, de matériaux d'isolation et analogues pour réduire la teneur en formaldéhyde de l'air environnant, dans laquelle les matériaux à base de bois
(i) contiennent une polyamine en tant que liant ou
(ii) contiennent un liant différent d'une polyamine et comportent également une polyamine dans ou sur les couches externes du matériau à base de bois,
la polyamine ayant un poids moléculaire d'au moins 500 g/mol et comportant au moins 6 groupes amino primaires ou secondaires.

2. Utilisation selon la revendication 1, dans laquelle la polyamine a un poids moléculaire d'au moins 800 g/mol et comporte au moins 10 groupes amino primaires ou secondaires.

3. Utilisation selon la revendication 1 ou 2, dans laquelle la polyvinylamine et/ou la polyéthylène imine est utilisée en tant que polyamine.

4. Utilisation selon la revendication 3, dans laquelle la polyvinylamine a un poids moléculaire moyen en poids de 5 000 à 500 000 g/mol et la polyéthylène imine un poids moléculaire moyen en poids de 500 à 100 000 g/mol.

5. Utilisation selon la revendication 3, dans laquelle la polyvinylamine a un poids moléculaire moyen en poids de 5 000 à 100 000 g/mol et la polyéthylène imine un poids moléculaire moyen en poids de 500 à 20 000 g/mol.

6. Utilisation de matériaux à base de bois selon les revendications 1 à 5 pour la fabrication de panneaux arrière de meubles.
